Europäisches Patentamt

European Patent Office

Office européen des brevets

(11) Publication number: **0 173 341**
**B1**

(12) EUROPEAN PATENT SPECIFICATION

(45) Date of publication of patent specification: **04.04.90**

(21) Application number: **85110914.0**

(22) Date of filing: **29.08.85**

(51) Int. Cl.⁵: **G 01 N 33/74,** G 01 N 33/76,
G 01 N 33/78, G 01 N 33/577
// C12N5/00, C12P21/00

(54) Method for the determination of hormones.

(30) Priority: **29.08.84 JP 181264/84**

(43) Date of publication of application:
**05.03.86 Bulletin 86/10**

(45) Publication of the grant of the patent:
**04.04.90 Bulletin 90/14**

(84) Designated Contracting States:
**AT BE CH DE FR GB IT LI NL SE**

(56) References cited:
**EP-A-0 014 965**
**EP-A-0 173 973**
**GB-A-2 111 201**
**US-A-3 992 514**

The file contains technical information
submitted after the application was filed and
not included in this specification

(73) Proprietor: **Juridical Foundation The Chemo-
Sero-Therapeutic Research Institute
668, Okubo Shimizu-machi
Kumamoto-shi Kumamoto-ken (JP)**

(72) Inventor: **Fujita, Haruo
1446-32, Kannabe-machi
Kumamoto-shi Kumamoto-ken (JP)**
Inventor: **Eda, Yasuyuki
Yoshino-so 203 2-82, Shimizuhigashi-machi
Kumamoto-shi Kumamoto-ken (JP)**
Inventor: **Nakashima, Toshihiro
7-733-32, Izumi
Kumamoto-shi Kumamoto-ken (JP)**
Inventor: **Unoki, Masanori
622-6, Shinchi Shimizu-machi
Kumamoto-shi Kumamoto-ken (JP)**
Inventor: **Tsuji, Ichiroh
5-18-27, Wakaba
Kumamoto-shi Kumamoto-ken (JP)**

(74) Representative: **Vossius & Partner
Siebertstrasse 4 P.O. Box 86 07 67
D-8000 München 86 (DE)**

## Description

The present invention relates to a method for the determination of one of the following hormones: thyroid-stimulating hormone (TSH), follicle-stimulating hormone (FSH), luteinizing hormone (LH) and human chorigonadotropin (HCG); wherein said method is based on an immunoassay technique by using monoclonal antibodies.

Determination of the amounts of these hormones contained in body fluids, particularly in blood, is extensively utilized in diagnosis of such disease as Graves' disease, cretinism or hypothyroidism, in which the determination is conducted by an immunoassay such as EIA (enzyme immunoassay) or RIA (radioimmunoassay) by using antibodies specific for the constituents of the hormones. Each of the hormones is constituted of its own α-subunit and β-subunit, in which the antigenicities of the α-subunits of the four hormones are quite similar to each other. Thus, conventional immunoassay techniques (EIA, RIA or the like), in which polyclonal antibodies are used, suffer from poor specificities and reproducibilities, due to the cross reactions between the hormones.

For the purpose of overcoming such disadvantage, new immunoassays have been proposed in which there are used monoclonal antibodies specific for the respective hormones. For example, "Clinical Chemistry, Vol. 28, No. 9, pp. 1862—1866, 1982" by H. G. Wada et al. discloses EIA which employs monoclonal antibodies specific for α-subunit and β-subunits of the above-mentioned hormones. More specifically, in the disclosed EIA, the anti-α-subunit monoclonal antibodies are used as the solid-phase antibodies (the capture antibodies) for the hormones and the anti-β-subunits monoclonal antibodies are coupled to a peroxidase to be used as the enzyme-labelled antibodies (the enzyme-antibody conjugate). Mention is made in H. G. Wada et al. that the anti-α-subunit monoclonal antibodies as the solid-phase can only be a monoclonal antibody specific for one of the α-subunits of the hormones because of the similarity in structures and immunochemical properties between the α-subunits of these hormones.

However, the experiments conducted by the present inventors in the manner disclosed by Wada et al. proved that such an anti-α-subunit monoclonal antibody does not necessarily exhibit the same degree of reactivity for all the hormones and considerably varies depending upon the clones from which the monoclonal antibodies are prepared. Thus, it is desired to prepare a monoclonal antibody which makes it possible to determine the amounts of the four hormones with substantially the same degree of sensitivity.

A primary object of the present invention is therefore to provide an improved immunoassay for the determination of one of the hormones TSH, FSH, LH and HCG which has high sensitivity due to the use of a monoclonal antibody.

For accomplishing the above object, the present inventors have made full studies of monoclonal antibodies, which can be derived from the fusion of spleen cells derived from BALB/c mice immunized with one of the hormones or its α-subunit and mouse myeloma cells, and found that such monoclonal antibodies are classified into several groups in terms of their reactivities with the hormones, depending upon the clones from which the antibodies are produced. For example, it has been found that monoclonal antibodies from the fusion of mouse spleen cells immunized with TSH (TSH-whole) or its α-subunit and myeloma cells can be classified into the following four groups:

(a) A monoclonal antibody having distinctly lower reactivity with LH than with the other hormones.

(b) A monoclonal antibody having distinctly higher reactivity with LH than with the other hormones.

(c) A monoclonal antibody having high reactivities with TSH and LH but low reactivities with HCG and FSH.

(d) A monoclonal antibody having high reactivities with all the four hormones.

This fact probably accounts for the presence of at least four epitopes in the α-subunit of the hormone. Thus, for determining the amounts of all the four hormones with high sensitivities it is essential to establish and use such a monoclonal as classified in (d).

The present invention is based on the above finding and provides a method for the determination of one of the hormones TSH, FSH, LH and HCG by means of an immunoassay, which comprises using, as the solid-phase antibody (the capture antibody) or as the labelled antibody (such as the enzyme-labelled antibody or the radioactively labelled antibody) for the assay, a monoclonal antibody directed to one of the four hormones or their α-subunit and having high reactivities with all the hormones.

The monoclonal antibody to be used in the present invention can be prepared by the fusion of spleen cells from BALB/c mice immunized with one of the four hormones or their α-subunit and myeloma cells. The preparation of monoclonal antibodies is carried out in a conventional manner known to those skilled in the art, as will be described below. Among monoclonal antibodies produced from various clones are selected monoclonal antibodies having high reactivities with all the hormones, which reactivities can be expressed in terms of the B/T value (as defined later) and are preferably at least 40% (with respect to the original solution which is also defined later) for the effective immunoassay.

The features of the present invention will be apparent from the following description.

Preparation of monoclonal antibodies:

A W/O (water in oil) suspension for use as antigen for primary immunization is prepared by dissolving 50 μg of TSH (TSH-whole) or the α-subunit of TSH in 0.9% physiological saline, and adding to the resultant solution 1.3 ml FCA (Freund Complete Adjuvant, as adjuvant) available from DIFCO Corp. A booster antigen is prepared by dissolving 50 μg of TSH (TSH-whole) or TSH α-subunit in 0.9% physiological saline.

Female BALB/c mice, at the ages of four to five weeks, are immunized in accordance with the schedule as shown in Table 1. The immunized mouse spleen cells are fused with mice myeloma cells (e.g. P3-X63-Ag8-U1) by means of the cell fusion technology for example, as shown in "Hybridoma Process and Monoclonal Antibodies" edited by T. Watanabe, pp. 20—29, R & D Planning Corp. (Japan), 1982 followed by cloning to obtain six monoclonal antibodies.

Table 1

Immunization Schedule

| Clone No. | Antigen | Primary Immunization | | Booster Immunization | | | Cell fusion |
|---|---|---|---|---|---|---|---|
| | | Amount | Injecton | Time of injection | Amount | Injection | |
| 1 | TSH-α | 5µg + FCA | i.p. | 48 days (after primary immunization) | 5 µg | i.v. | Four days after Booster immunization |
| 2 | TSH | do. | do. | 60 days | 10 µg | do. | do. |
| 3 | TSH | do. | do. | 48 days | 5 µg | do. | do. |
| 4 | TSH | do. | do. | do. | do. | do. | do. |
| 5 | TSH-α | do. | do. | do. | do. | do. | do. |
| 6 | TSH | do. | do. | 60 days | 10 µg | do. | do. |

Clonal specificities of the six monoclonal antibodies were determined with the RIA method by using [125]I-labelled TSH, [125]I-labelled α-subunit TSH and [125]I-labelled β-subunit TSH as the tracers, with the results given in Table 2, in which the values for the specificities are expressed in cpm as compared with the negative controls of the culture supernatants from the mouse myeloma cells (P3-X63-Ag8-U1).

## Table 2

| Clone | Tracer | | | Antigenic |
|---|---|---|---|---|
| No. | TSH-whole | α | β | Specificity |
| 1 | 4.9 | 21.2 | 1.0 | α |
| 2 | 6.2 | 32.1 | 1.1 | α |
| 3 | 3.3 | 6.6 | 1.0 | α |
| 4 | 6.5 | 33.8 | 1.0 | α |
| 5 | 5.7 | 17.7 | 1.4 | α |
| 6 | 9.3 | 42.1 | 1.2 | α |

The monoclonal antibodies were further determined with respect to the reactivities with the four hormones, the association constants to TSH, and the immunoglobulin subclass.

In determining the reactivities, an original solution for each monoclonal antibody was prepared by adjusting the immunoglobulin concentration in the culture supernatant of the clone to be 20 µg/ml. 100 µg of such original solution for each monoclonal antibody and 100 µl of its dilute solution (diluted five times) are reacted with [125]I-labelled TSH, [125]I-labelled HCG, [125]I-LH or [125]I-FSH as the tracer (each 100 µl) to determine the reactivities (B) expressed in cpm (counts per minute), as shown in Table 3. In the table are also provided the ratios B/T, in which T designates the total counts (cpm) of each tracer (100 µl).

The association constants of the monoclonal antibodies were obtained by examining the ascites fluids containing the clones, as follows in the known manner, for example, as described in "Experimental procedures in immunology IX — Determination of association constant of antibodies" published by Immunological Society of Japan on December 4, 1980, pages 269—2705.

The monoclonal antibodies were also determined with respect to the immunoglobulin-subclass, through Ouchterlony's method, by examining the culture supernatants from the respective clones, by use of antiserum containing antibodies against mouse-α, $-\gamma_1$, $-\gamma_2^a$, $-\gamma_2^b$, $-\gamma_3$, -µ, -κ or -λ chain.

The results are summarized in Table 3. It is particularly noted that there were obtained monoclonal antibodies having different reactivities which can be classified into the four groups as described previously. Of the monoclonal antibodies obtained, the antibody of clone No. 6 has high reactivities with all the four hormones, with all the B/T values being at least 40%, and belongs to the group (d). Notably clone No. 6 appeared to be of an immunoglobulin subclass which is quite similar to IgM, while all the other clones are of IgC immunoglobulin subclass.

Table 3

| Clone No. | | Reactivity with each hormone | | | | | | | | Reactivity group | Association constant (liters/mole) | Immunoglobulin subclass | |
|---|---|---|---|---|---|---|---|---|---|---|---|---|---|
| | | TSH | | HCG | | LH | | FSH | | | | | |
| | | Original solution | 1/5 diluted | Original solution | 1/5 diluted | Original solution | 1/5 diluted | Original solution | 1/5 diluted | | | H chain | L chain |
| 1 | B(cpm) | 5960 | 4820 | 5592 | 5249 | 1657 | 1204 | 4267 | 3080 | a | $4.5 \times 10^8$ | γ1 | κ |
| | B/T(%) | 29.2 | 23.6 | 28.8 | 27.1 | 9.2 | 6.7 | 23.0 | 16.6 | | | | |
| 2 | B(cpm) | 5347 | 3882 | 5764 | 4460 | 1310 | 906 | 4164 | 2845 | a | $4.0 \times 10^8$ | γ1 | κ |
| | B/T(%) | 26.2 | 19.0 | 29.7 | 23.0 | 7.2 | 5.0 | 22.4 | 15.3 | | | | |
| 3 | B(cpm) | 3532 | 1769 | 5222 | 3882 | 10539 | 8485 | 1784 | 731 | b | $3.0 \times 10^8$ | γ1 | κ |
| | B/T(%) | 17.3 | 8.6 | 26.9 | 20.0 | 58.3 | 46.9 | 9.6 | 3.9 | | | | |
| 4 | B(cpm) | 9933 | 8387 | 6131 | 4948 | 10754 | 9179 | 4824 | 3528 | c | $3.8 \times 10^9$ | γ1 | κ |
| | B/T(%) | 48.6 | 41.0 | 31.6 | 25.5 | 59.5 | 50.8 | 26.0 | 19.0 | | | | |
| 5 | B(cpm) | 9093 | 7273 | 4890 | 4152 | 8569 | 6348 | 3654 | 2925 | c | $6.1 \times 10^9$ | γ1 | κ |
| | B/T(%) | 44.5 | 35.6 | 25.2 | 21.4 | 47.4 | 35.1 | 19.7 | 15.8 | | | | |
| 6 | B(cpm) | 8605 | 6770 | 7819 | 6481 | 9307 | 8123 | 8500 | 5825 | d | $1.2 \times 10^9$ | similar to μ | κ |
| | B/T(%) | 42.1 | 33.1 | 40.3 | 33.4 | 51.5 | 44.9 | 45.8 | 31.4 | | | | |
| Negative Control Total(cpm) | | 875 20438 | | 910 19403 | | 654 18076 | | 408 18556 | | | | | |

EP 0 173 341 B1

EP 0 173 341 B1

Example

EIA experiments were carried out by using the six clones (monoclonal antibodies) prepared in the manner as described above.

A standard solution (200 µl) having a predetermined concentration of one of the hormones and polystyrene beads coated with one of said monoclonal antibodies (the solid-phase) are placed in a tube so as to make the reaction therebetween at 37°C for 2 hours. The beads were then washed three times with 5% Tween 20-PBS. There was added 200 µl of an enzyme-labelled anti-β-subunit antibody for the reaction at 37°C for 2 hours, followed by washing three times with 5% Tween 20-PBS.

To the washed product was added 500 µl of a solution which has been obtained by dissolving 25 µl of 5% $H_2O_2$ and 15 mg of o-phenylenediamine·2HCl in 25 ml solution containing 0.1M citric acid and 0.2M sodium phosphate, so as to carry out the reaction at 37°C for one hour in dark. The reaction was terminated by the addition of 125 µl of 6N $H_2SO_4$. A standard calibration curve for each assay system was obtained by plotting the optical densities (O.D.) of the reaction products (in terms of absorbances at 492 nm) against the concentration of each hormone. As the sensitivity of each assay system was regarded the concentration value corresponding to the O.D. at the zero concentration multiplied by 2.1. Thus, the value signifies the minimum concentration which can be detected by the assay system. The results are summarized in Table 4.

The hormones used in the preparation of the standard solutions were those available from Calbiochem (U.S.A.) and had the following activities:

| Hormone | Activity |
|---------|----------|
| TSH | 5 I.U./mg |
| HCG | 6,610 I.U./mg |
| LH | 10,500 I.U./mg |
| FSH | 5,800 I.U./mg |

## Table 4

## Assay sensitivities (in ng/ml)

| Clone No. | TSH | HCG | LH | FSH |
|-----------|-----|-----|-----|-----|
| 1 | 0.1 – 0.2 | 0.2 | 1.6 | 0.2 – 0.4 |
| 2 | 0.1 – 0.2 | 0.2 | 1.6 – 3.2 | 0.2 – 0.4 |
| 3 | 0.4 | 0.2 – 0.4 | 0.025 | 1.6 |
| 4 | 0.025 | 0.2 | 0.025 | 0.2 |
| 5 | 0.025 | 0.2 – 0.4 | 0.05 | 0.4 |
| 6 | 0.025 | 0.05 | 0.05 | 0.05 |

As can be seen from the results given in Table 4, the use of the clone No. 6 provides an immunoassay which makes it possible to determine each of the four hormones with very high sensitivities. Thus, according to the present invention there is provided a method for determining one of the four hormones by using, as the antibody bound to the solid phase or as the labelled antibody, the specific monoclonal antibody as described in detail above, while using, as the labelled antibody, or as the antibody bound to the solid phase, an antibody (not necessarily a monoclonal antibody) which is specific for the β-subunit of the hormone to be measured. Examples of such anti-β-subunit monoclonal antibodies are described in the applicant's co-pending European application 0173973 entitled "Method for determination of TSH". Further, anti-β-subunit monoclonal antibodies as described in H. G. Wada et al. can also be used.

The method of the present invention is not restricted to EIA as described in the Example, but can be applied also to other immunoassays (e.g. RIA) as well.

7

# EP 0 173 341 B1

**Claims**

1. A method for the determination of one of the hormones thyroid-stimulating hormone, follicle-stimulating hormone, luteinizing hormone or human chorigonadotropin by means of an immunoassay, comprising the following steps:

(a) contacting an antibody bound to a solid phase with the sample to be tested which potentially contains one of said hormones as an antigen, whereby an antigen-antibody complex is formed, and washing the solid phase in order to remove the residue of the sample;

(b) contacting the antigen-antibody complex obtained in (a) which is bound to the solid phase with a labelled antibody, whereby the labelled antibody is bound to the antigen of the antigen-antibody complex, and washing the solid phase in order to remove the unreacted labelled antibody; and

(c) determining the amount of the labelled antibody bound in (b) in order to measure the amount of the hormone contained in the sample;

characterized in that the antibody bound to the solid phase or the labelled antibody is a monoclonal antibody produced by a hybridoma derived from spleen cells of an animal immunized with one of said hormones or their α-subunits and having a reactivity with all of said hormones of at least 40% as expressed by the B/T value, and in that the other antibody is an antibody which is specific for the β-subunit of the hormone or hormones to be detected.

2. The method according to Claim 1, in which the monoclonal antibody is produced by a hybridoma prepared by fusing spleen cells from BALB/c mice immunized with one of said hormones or their α-subunits with myeloma cells.

3. The method according to Claim 1 or 2, in which the monoclonal antibody is produced by a hybridoma prepared by fusing spleen cells from BALB/c mice immunized with the thyroid-stimulating hormone or its α-subunit with myeloma cells.

**Patentansprüche**

1. Verfahren zur Bestimmung eines der folgenden Hormone, Thyroid stimulierendes Hormon, Follikel stimulierendes Hormon, luteinisierendes Hormon oder menschliches Chorigonadotropin mittels eines Immunoassays, umfassend die folgenden Stufen:

(a) Zusammenbringen eines an eine feste Phase gebundenen Antikörpers mit der zu testenden Probe, die möglicherweise eines der Hormone als ein Antigen enthält, wobei ein Antigen-Antikörper-Komplex gebildet wird, und Waschen der festen Phase zur Entfernung der restlichen Probe;

(b) Zusammenbringen des in (a) erhaltenen Antigen-Antikörper-Komplexes, der an die feste Phase gebunden ist, mit einem markierten Antikörper, wobei der markierte Antikörper an das Antigen des Antigen-Antikörper-Komplexes gebunden wird, und Waschen der festen Phase zur Entfernung von nichtumgesetzten markierten Antikörpern; und

(c) Bestimmung der Menge des in (b) gebundenen markierten Antikörpers zur Messung der Menge des in der Probe enthaltenen Hormons,

dadurch gekennzeichnet, daß der an die feste Phase gebundene Antikörper oder der markierte Antikörper ein monoclonaler Antikörper ist, der durch ein Hybridom erzeugt wurde, das von Milzzellen eines mit einem der genannten Hormone oder ihren α-Untereinheiten immunisiert worden ist, und der eine Reaktivität mit all den genannten Hormonen von mindestens 40%, ausgedrückt durch den B/T-Wert aufweist, und daß der andere Antikörper ein Antikörper ist, der spezifisch für die β-Untereinheit des oder der nachzuweisenden Hormone ist.

2. Verfahren nach Anspruch 1, in dem der monoclonale Antikörper durch ein Hybridom erzeugt wird, daß durch Verschmelzen von Milzzellen von Balb/c-Mäusen, die mit einem der genannten Hormone oder ihren α-Untereinheiten immunisiert wurden, mit Myeloma-Zellen hergestellt worden ist.

3. Verfahren nach Anspruch 1 oder 2, wobei der monoclonale Antikörper durch ein Hybridom erzeugt wird, das durch Verschmelzen von Milzzellen von Balb/c-Mäusen, die mit dem Tyroid stimulierenden Hormon oder seiner α-Untereinheit immunisiert wurden, mit Myeloma-Zellen hergestellt worden ist.

**Revendications**

1. Un procédé de dosage de l'une des hormones suivantes, hormone de stimulation de la glande thyroïde, hormone de stimulation de la follicule, hormone lutéinisante, ou chorigonadotropine humaine, par dosage immunologique, comprenant les étapes suivantes:

a) mise en contact d'une anticorps lié à une phase solide avec l'échantillon à tester qui contient potentiellement l'une desdites hormones en tant qu'antigène, de sorte qu'il se forme un complexe antigène-anticorps, et lavage de la phase solide en vue d'éliminer le résidu de l'échantillon;

b) mise en contact du complexe antigène-anticorps obtenu dans (a) qui est lié à la phase solide avec un anticorps marqué, de sorte que l'anticorps marqué est lié à l'antigène du complexe antigène-anticorps, et lavage de la phase solide pour éliminer l'anticorps marqué n'ayant pas réagi; et

c) dosage de la quantité de l'anticorps marqué lié dans (b) afin de mesurer la quantité de l'hormone contenue dans l'échantillon;

8

caractérisé en ce que l'anticorps lié à la phase solide ou l'anticorps marqué est un anticorps monoclonal produit par un hybridome dérivé des cellules de la rate d'un animal immunisé avec l'une de ces hormones ou leurs α-sous-unités et ayant une réactivité avec toutes ces hormones d'au moins 40%, exprimée par la valeur B/T, et en ce que l'autre anticorps est un anticorps qui est spécifique de la β-sous-unité de l'hormone ou des hormones à détecter.

2. Le procédé selon la revendication 1, selon lequel l'anticorps monoclonal est produit par un hybridome préparé par fusion des cellules de la rate provenant d'une souris BALB/c immunisée par une desdites hormones ou leurs α-sous-unités avec les cellules du myélome.

3. Le procédé selon la revendication 1 ou 2, selon lequel l'anticorps monoclonal est produit par un hybridome préparé par fusion des cellules de la rate de la souris BALB/c immunisée avec l'hormone de stimulation de la thyroïde ou son α-sous-unité avec les cellules du myélome.